## Europäisches Patentamt

## European Patent Office

## Office européen des brevets

(11) Publication number: **0 103 915**
**B1**

(12) **EUROPEAN PATENT SPECIFICATION**

(45) Date of publication of patent specification: **09.12.87**

(21) Application number: **83201089.6**

(22) Date of filing: **25.07.83**

(51) Int. Cl.⁴: **C 12 Q 1/00,** G 01 N 31/22, G 01 J 3/46

(54) Improved visual reading of colour tests.

(30) Priority: **17.08.82 NL 8203221**

(43) Date of publication of application:
**28.03.84 Bulletin 84/13**

(45) Publication of the grant of the patent:
**09.12.87 Bulletin 87/50**

(84) Designated Contracting States:
**DE FR GB NL**

(56) References cited:
**GB-A-1 186 668**
**NL-A-8 001 972**
**US-A-4 340 394**

**RESEARCH DISCLOSURE, no. 160, August 1977, pages 19-24, Hampshire, GB; "Compositions for the detection of hydrogen peroxide"**

**TETRAHEDRON, vol. 30, 1974, pages 3299-3302, Pergamon Press, Oxford, GB; V.R.HOLLAND et al.: "A safer substitute for benzidine in the detection of blood"**

(73) Proprietor: **AKZO N.V.**
**Velperweg 76**
**NL-6824 BM Arnhem (NL)**

(72) Inventor: **Kuypers, Leonardus Paulus Clement**
**Gelissenstraat 24**
**Oss (NL)**
Inventor: **Gribnau, Thomas Cornelis Josephus**
**Berghemseweg 4**
**Haaren (NL)**

(74) Representative: **Douma, Anno Dominicus et al**
**Postbus 20**
**NL-5340 BH Oss (NL)**

(56) References cited:
**CLINICAL CHEMISTRY, vol. 28, no. 4, April 1982, pages 578-588, Washington, US; R.H.WHITE-STEVENS: "Interference by ascorbic acid in test systems involving peroxidase. I.Reversible idicators and the effects of copper, iron, and mercury"**

**Academic Press Inc. New York Chemistry of Synthetic Dyes, Vol. II, 1952, pages 1347-1348, K. Venkataraman**

Courier Press, Leamington Spa, England.

# 0 103 915

**Description**

The invention relates to a method for the visual determination of the intensity of the colour of a liquid in a series of vessels containing different dyestuff concentrations, after having carried out an immunochemical determination in said series of vessels giving rise to a colouration or colour change of the reaction medium. The invention further relates to test kits for use in said method.

Visual determination of the colour intensity of a liquid is, by virtue of its simplicity and rapidity, an attractive method for among others detection and/or concentration determination of substances in a test medium. Such visual determinations are carried out as a matter of routine, in very large numbers, in numerous laboratories.

A substantial problem in this context is, however, that, depending on the dyestuff in the solution, the eye is relatively insensitive to the frequently only very slight colour intensity differences. This has an adverse effect on the reliability of such a determination.

The object of the process according to the invention is to make such methods of determination substantially more reliable. In The Chemistry of Synthetic Dyes, Volume II, 1952, Academic Press Inc. New York, pages 1347—1348 by K. Venkataraman a colorimetric estimation of dyestuffs is disclosed by comparing the colour of the solution under test with the colour of a standard solution of the same dye while passing light through the solutions, which light illuminates the two halves of an eyepiece field. These two halves are equalized by altering the depths of the solutions. The concentration of the solutions are then inversely proportional to the depths, provided Beer's law is obeyed. For more accurate results, especially with weakly coloured solutions, light of a narrow range of wavelength, corresponding to the main absorption bond of the dye, may be employed. This reference is not concerned with the use of non-complementary dyestuffs for improving the above result, nor with immunochemical determinations. Further, as far as this reference is concerned with visual detection, such detection is related to visual matching followed by the use of a colorimeter.

Surprisingly, it has been found that visual reading of immunochemical colour tests is made more reliable by carrying out the visual determination in the presence of at least one additional dyestuff wihh a non-complementary colour.

Accordingly, the present invention relates to a method for the visual determination of the intensity of the colour of a liquid in a series of vessels containing different dyestuff concentrations, after having carried out an immunochemical determination in said series of vessels giving rise to a colouration or colour change of the reaction medium, characterized in that the determination is carried out in the presence of one or more additional dyestuffs with a colour which is non-complementary to that of the first dyestuff, so that, in the vessels containing different concentrations of the first dyestuff a different colour or colour shade is produced.

Further the invention relates to a test kit for use in said method, characterized in that the test kit contains, in addition to the conventional components for immunochemical determination, a series of transparent coloured vessels having a colour different from and non-complementary to the colour which is formed as a result of the particular immunochemical determination or that the test kit contains, in addition to the conventional components for immunochemical determinations, a dyestuff having a colour different from and non-complementary to the colour which is formed as a result of the particular immunochemical determination or that the test kit contains, in addition to the conventional components for immunochemical determinations, one or more translucent or transparent sheets having a colour different from and non-complementary to the colour which is formed as a result of the particular immunochemical determination.

Comparison of the resulting colour with others in the series of vessels, including blanks and standards, permits a qualitative and/or semi-quantitative detection of the substance to be determined.

According to the invention it is possible to incorporate the extra dyestuff, or—if several are present—one or more of the extra dyestuffs, in the material from which the vessels for carrying out the determination are produced.

Another possibility is to add an extra dyestuff, or several of the extra dyestuffs, to the liquid in the vessels. The dyestuff(s) can be in true solution, or e.g. in dispersed form.

It is also possible, when using several extra dyestuffs, to add one of these to the liquid and another to the material from which the vessels for carrying out the determination are produced. The dyestuff or dyestuffs can also be incorporated in a transparent or translucent sheet through which light striking the vessels is transmitted.

The extra dyestuff added in accordance with the invention is non-complementary, resulting in colour differences when there are concentration differences of the first dyestuff. Preferably yellow and cyan are employed as non-complementary colours.

An immunochemical reaction which, in the method according to the invention, can change the colour intensity may comprise the addition of a compound which reacts with the substance to be determined, but can also comprise a plurality of addition steps, possible intermediate processing as well as heating, cooling, crystallisation and adsorption, as well as the addition of reactants which are later removed again.

Immunochemical determinations wherein a colour reaction occurs, are those wherein a dye or metal sol particles are used, but in particular those determinations where detection takes place through the

2

# 0 103 915

measurement of enzyme activity with the aid of a substrate, which upon conversion leads to a formation or change of colouration.

The routine performing of immunochemical determinations frequently employs ready-to-use sets which comprise reagents, reaction vessels and auxiliaries, and which are referred to as test kits. For using the method according to the invention, special test kits can be employed.

These test kits for example comprise, in addition to the conventional components for the immunochemical determination, a series of coloured vessels, a translucent or transparent coloured sheet or a loose added dyestuff or a plurality of dyestuffs having a colour different from that of the liquid which is formed as a result of the particular immunochemical determination.

Depending on the type of immunochemical determination, a test kit for example includes, as particular conventional components, a labelled immuno component, an immuno component optionally bonded to a carrier, wash liquid and/or buffer.

A test kit for enzyme immuno-assays according to the invention comprises one oc more enzyme-coupled immuno components, a substrate for the coupled enzyme and a chromogen, as well as a series of coloured vessels, a translucent or transparent coloured sheet or a loose added dyestuff or a plurality of dyestuffs having a colour different from that of the liquid which was formed as a result of the particular immunochemical determination.

The invention is illustrated with the aid of the examples which follow.

## Example 1

The enzyme activity of samples containing horseradish peroxidase is determined by incubation of the samples in buffer (0.01 mol of sodium acetate/litre; adjusted to pH 6.0 with solid citric acid) to which substrate ($H_2O_2$; 20 mmoles/litre) and chromogen (3,3', 5,5'-tetramethylbenzidine, "TMB"; 0.4 mmol/litre) are added. After incubation (1 hour at room temperature) the reaction is stopped by adding sulphuric acid (2 mole/litre).

The enzyme activity present ultimately results in a yellow colour (absorption maximum: 450 nm), with differences in activity between samples manifesting themselves as differences in colour intensity. The differentiation between these in the case of a "+/−" reading, for example made visually, is accentuated by converting the intensity difference into a colour change. This is done by adding to the sulphuric acid solution (see above) the blue dyestuff Toluidine Blue O; concentration corresponding to $A^1_{596}cm=1.860$. The series of "low to high intensity of yellow" (or: light yellow to dark yellow) is thus changed to a series varying from blue via bluish green/greenish yellow to yellow.

| Enzyme activity | Without Toluidine Blue | With Toluidine Blue |
|---|---|---|
| — | colourless/light yellow | blue |
| +...+++ | light yellow/dark yellow | bluish green/greenish yellow |
| ++++ | dark yellow | yellow |

## Example 2

The determination according to Example 1 was carried out, except that the reaction was in this case not stopped with sulphuric acid.

Enzyme activity present now results in a blue colour (absorption maximum: 655 nm); activity differences between samples manifest themselves as different intensities of blue. The differences again become more clearly readable by converting the intensity difference into a colour change, namely by adding a solution of the yellow dyestuff Primazin$^R$ Yellow GRL, BASF in distilled water; concentration corresponding to $A^1_{415}cm=1.820$.

| Enzyme activity | Without Primazin$^R$ Yellow | With Primazin$^R$ Yellow |
|---|---|---|
| — | colourless/light blue | yellow |
| +...+++ | light blue/dark blue | yellowish green/bluish green |
| ++++ | dark blue | blue |

## Example 3

The determination according to Example 2 was carried out, except that N,N-dimethyl-p-phenylenediamine dihydrochloride was used as a chromogen in place of TMB. Enzyme activity present

3

now results in red colour (absorption maximum: 520 nm). The conversion of the colour intensity difference into a colour change is achieved by adding a solution of the blue dyestuff Toluidine Blue Ö in distilled water (see Example 1).

| Enzyme activity | Without Toluidine Blue | With Toluidine Blue |
|---|---|---|
| — | colourless/light red | blue |
| +...+++ | light red/dark red | reddish violet/purple |
| ++++ | dark red | red |

Example 4

In a Sandwich enzyme immuno-assay for Hepatitis B surface antigen (Hepanostika[R] HBsAg: Organon Teknika) use is made of an antibody labelled with horseradish peroxidase. The presence of the antigen in the blood sample to be investigated results in the bonding of a quantity of enzyme to the well in a polystyrene microtitration plate; the enzyme activity is rendered visible by incubation with buffer/substrate $(H_2O_2)$/chromogen (TMB), followed by addition of sulphuric acid. The "positive/negative" result is now determined by visual comparison of the colour of the wells containing sample with those of the wells with positive and negative control. The reading is greatly simplified by following the procedure described in Example 1. The positive samples "stand out" as greenish yellow/yellow amongst the blue negative samples.

**Claims**

1. Method for the visual determination of the intensity of the colour of a liquid in a series of vessels containing different dyestuff concentrations, after having carried out an immunochemical determination in said series of vessels giving rise to a colouration or colour change of the reaction medium, characterized in that the determination is carried out in the presence of one or more additional dyestuffs with a colour which is non-complementary to that of the first dyestuff, so that, in the vessels containing different concentrations of the first dyestuff a different colour or colour shade is produced.

2. Method according to claim 1, characterized in that transparent vessels produced from a material wherein at least one dyestuff is included are employed.

3. Method according to claim 1, characterized in that one or more extra dyestuff(s) is or are added to the liquid in the vessels.

4. Method according to claim 1, characterized in that at least two extra dyestuffs are employed, with at least one dyestuff being included in the material from which the vessels are produced and at least one other extra dyestuff being added to the liquid in the vessels.

5. Method according to claim 1, characterized in that yellow and cyan are employed as non-complementary colours.

6. Method according to claim 1, characterized in that the determination is an enzyme immunochemical determination.

7. Test kit for use in the method according to claims 1 or 6 for the visual determination of the intensity of the colour of a liquid in a series of vessels containing different dyestuff concentrations, characterized in that the test kit contains, in addition to the conventional components for immunochemical determination, a series of transparent coloured vessels having a colour different from and non-complementary to the colour which is formed as a result of the particular immunochemical determination.

8. Test kit for use in the method according to claims 1 or 6 for the visual determination of the intensity of the colour of a liquid in a series of vessels containing different dyestuff concentrations, characterized in that the test kit contains, in addition to the conventional components for immunochemical determinations, a dyestuff having a colour different from and non-complementary to the colour which is formed as a result of the particular immunochemical determination.

9. Test kit for use in the method according to claims 1 or 6 for the visual determination of the intensity of the colour of a liquid in a series of vessels containing different dyestuff concentrations, characterized in that the test kit contains, in addition to the conventional components for immunochemical determinations, one or more translucent or transparent sheets having a colour different from and non-complementary to the colour which is formed as a result of the particular immunochemical determination.

**Patentansprüche**

1. Verfahren zur visuellen Bestimmung der Farbintensität einer Flüssigkeit in einer Reihe von verschiedene Farbstoffkonzentrationen enthaltenden Gefässen nach Durchführung einer immunchemischen Bestimmung in dieser Reihe von Gefässen, wobei eine Färbung oder ein Farbumschlag des Reaktionsmediums eintritt, dadurch gekennzeichnet, dass man die Bestimmung in Gegenwart eines oder mehrerer weiterer Farbstoffe einer Fabre durchführt, die zu derjenigen des ersten Farbstoffs nicht komplementär ist, so dass in den verschiedene Konzentrationen des ersten Farbstoffs enthaltenden Gefässen eine unterschiedliche Farbe bzw. Farbton entsteht.

2. Verfahren nach Anspruch 1, dadurch gekennzeichnet, dass man dabei durchsichtige, aus einem mindestens einen Farbstoff mitenthaltenden Material hergestellte Gefässe verwendet.

3. Verfahren nach Anspruch 1, dadurch gekennzeichnet, dass man einen oder mehrere weitere Farbstoff(e) der Flüssig keit in dén Gefässen zusetzt.

4. Verfahren nach Anspruch 1, dadurch gekennzeichnet, dass man mindestens zwei weitere Farbstoffe einsetzt, wobei mindestens ein Farbstoff in dem die Gefässe bildenden Material eingeschlossen ist und mindestens ein weiterer Farbstoff zusätzlich in die Flüssigkeit in den Gefässen gegeben wird.

5. Verfahren nach Anspruch 1, dadurch gekennzeichnet, dass man gelb und blaugrün als die nicht-komplementären Farben verwendet.

6. Verfahren nach Anspruch 1, dadurch gekennzeichnet, dass die Bestimmung eine enzym immunchemische Bestimmung ist.

7. Testsatz zum Gebrauch bei dem Verfahren nach Anspruch 1 oder 6 zur visuellen Bestimmung der Farbintensität einer Flüssigkeit in einer Reihe von verschiedene Farbstoffkonzentrationen enthaltenden Gefässen, dadurch gekennzeichnet, dass er zusätzlich zu den üblichen Komponenten für immunchemische Bestimmung eine Reihe von durchsichtigen farbigen Gefässen enthält, deren Färbung sich von der als Ergebnis der jeweiligen immunchemischen Bestimmung gebildeten Farbe unterscheidet und mit dieser nicht komplementär ist.

8. Testsatz zum Gebrauch bei dem Verfahren nach Anspruch 1 oder 6 zur visuellen Bestimmung der Farbintensität einer Flüssigkeit in einer Reihe von verschiedene Farbstoffkonzentrationen enthaltenden Gefässen, dadurch gekennzeichnet, dass er zusätzlich zu den üblichen Komponenten für immunchemische Bestimmungen einen Farbstoff enthält, dessen Färbung sich von der als Ergebnis der jeweiligen immunchemischen Bestimmung gebildeten Farbe unterscheidet und mit dieser nicht komplementär ist.

9. Testsatz zum Gebrauch bei dem Verfahren nach Anspruch 1 oder 6 zur visuellen Bestimmung der Farbintensität einer Flüssigkeit in einer Reihe von verschiedene Farbstoffkonzentrationen enthaltenden Gefässen, dadurch gekennzeichnet, dass er zusätzlich zu den üblichen Komponenten für immunchemische Bestimmung eine oder mehrere durchscheinende oder durchsichtige Folien enthält, deren Färbung sich von der als Ergebnis der jeweiligen immunchemischen Bestimmung gebildeten Farbe unterscheidet und mit dieser nicht komplementär ist.

## Revendications

1. Méthode pour la détermination visuelle de l'intensité de la couleur d'un liquide contenu dans une série de récipients contenant différentes concentrations de matière colorants, après qu'ait été effectuée dans ladite série de récipients une épreuve immunochimique donnant naissance à une coloration ou un changement de couleur du milieu de réaction, caractérisée par le fait que la détermination est effectuée en présence d'une on plusieurs matières colorantes supplémentaires ayant une couleur non complémentaire de celle de la première matière colorante, de sorte que soit engendrée une couleur ou nuance de couleur différente dans les récipients contenant différentes concentrations de la première matière colorant.

2. Méthode selon la revendication 1, caractérisée par le fait que l'on utilise des récipients transparents faits d'une matière dans laquelle est incorporée au moins une matière colorante.

3. Méthode selon la revendication 1, caractérisée par le fait qu'une ou plusieurs matières colorantes supplémentaires sont ajoutées au liquide contenu dans les récipients.

4. Méthode selon la revendication 1, caractérisée par le fait qu'on utilise au moins deux matières colorantes supplémentaires, l'une au moins des matières colorantes étant incorporée dans la matière dont sont faits les récipients et au moins une autre des matières colorantes supplémentaires étant ajoutée au liquide contenu dans les récipients.

5. Méthode selon la revendication 1, caractérisée par le fait qu'on utilise le jaune et le cyan comme couleurs non complémentaires.

6. Méthode selon la revendication 1, caractérisée par le fait que l'épreuve est une épreuve immunochimique enzymatique.

7. Kit d'essai à utiliser dans la méthode selon les revendications 1 ou 6 pour la détermination visuelle de l'intensité de la couleur d'un liquide contenu dans une série de récipients contenant différentes concentrations de matière colorants, caractérisé par le fait que le kit d'essai contient, en plus des composants classiques pour une épreuve immunochimique, une série de récipients transparents colorés ayant une couleur différente et non complémentaire de la couleur qui est engendrée comme résultat de l'épreuve immunochimique particulière.

8. Kit d'essai à utiliser dans la méthode selon les revendications 1 ou 6 pour la détermination visuelle de l'intensité de la couleur d'un liquide contenu dans une série de récipients contenant différentes concentrations de matière colorants, caractérisé par le fait que le kit d'essai contient, en plus des composants classiques pour une épreuve immunochimique, une matière colorante ayant une couleur

**0 103 915**

différente et non complémentaire de la couleur qui est engendrée comme résultat de l'épreuve immunochimique particulière.

9. Kit d'essai à utiliser dans la méthode selon les revendications 1 ou 6 pour la détermination visuelle de l'intensité de la couleur d'un liquide contenu dans une série de récipients contenant différentes concentrations de matière colorante, caractérisé par le fait que le kit d'essai contient, en plus des composants classiques pour une détermination immunochimique, une ou plusieurs feuilles transparentes ou translucides ayant une couleur différente et non complémentaire de la couleur qui est engendrée comme résultat de l'épreuve immunochimique particulière.

6